# DEMANDE DE BREVET EUROPEEN

(11) **EP 0 524 857 A1**
(43) Date de publication de la demande: **27.01.1993**
(21) Numéro de dépôt: 92401984.7
(22) Date de dépôt: 09.07.1992
(51) Int. Cl.: F16C 41/04, F16C 19/54, A61F 2/36

(54) **Roulement combiné radial axial**

(30) Priorité: 26.07.1991 FR 9109491
(71) Demandeur: S.N.R. ROULEMENTS, F-74010 Annecy Cédex (FR)
(72) Inventeur: Hajzler, Christian, F-74000 - Annecy (FR); Coux, Isabelle, F-74600 - Seynod (FR)
(74) Mandataire: Ernst-Schonberg, Michel

(57) **Abrégé**

Roulement combiné radial-axial à corps roulants (3, 4) montés sur une bague intérieure (1) dans lequel un boîtier extérieur (2) de réception possède une première surface périphérique intérieure (6) qui constitue le chemin de roulement des corps roulants (3) de support des contraintes radiales et une deuxième surface (7) sensiblement perpendiculaire qui constitue le chemin de roulement des corps roulants (4) de support des contraintes axiales, montés dans une cage (5) de réception et de répartition des corps roulants, constituée par un anneau extérieur et un anneau intérieur concentriques et des barres de liaison et d'espacement radiales, caractérisé par le fait que l'anneau extérieur de la cage possède une face radiale axialement décalée par rapport à la face radiale correspondantes de l'anneau intérieur et que lesdites faces délimitent un espace (10) de rangement d'un organe (11) de maintien transitoire de corps roulants de support des contraintes radiales à la suite du déplacement axial relatif du boîtier extérieur (2) par rapport à la bague intérieure (1).

## Description

L'invention concerne un roulement combiné radial-axial à corps roulants montés sur une bague intérieure dans lequel un boîtier extérieur de réception possède une première surface périphérique intérieure qui constitue le chemin de roulement des organes roulants de support des contraintes radiales et une deuxième surface sensiblement perpendiculaire qui constitue le chemin de roulement des organes roulants de support des contraintes axiales.

La publication FR-A-2167551 décrit un tel roulement.

Un exemple d'application de ces roulements peut-être trouvé dans le domaine des prothèses de hanche. Dans ce type d'application, la tête de la prothèse subit des charges qui sont absorbées par un roulement radial-axial.

La publication FR-A-2584603 décrit une prothèse dont la tête subit des charges absorbées par une bague intérieure en une ou deux parties qui porte les chemins de roulement des corps roulants.

L'invention s'applique plus particulièrement aux roulements combinés dans lesquels les corps roulants sont placés entre un boîtier extérieur et une bague intérieure.

Lorsque le roulement est soumis à des charges importantes il s'est avéré avantageux d'augmenter le nombre des corps roulants et de supprimer l'une des cages de répartition correspondante.

Une telle disposition ne facilite pas l'assemblage du roulement lorsque la rangée des corps roulants sans cage de rétention doit supporter l'effort radial et est disposée dans un boîtier extérieur latéralement fermé qui contient la rangée de corps roulants qui supporte l'effort axial.

L'invention a pour objet un roulement assemblé dans lequel un organe de maintien mobile coopère fonctionnellement à la cohésion transitoire des corps roulants pendant le montage et le déplacement axial relatif du boîtier par rapport à la bague.

L'invention a également pour objet un roulement assemblé dans lequel l'organe de maintien des corps roulants occupe une position de rangement dans la cage de répartition d'une rangée de corps roulants.

Selon un aspect de l'invention l'anneau extérieur de la cage possède une face radiale axialement décalée par rapport à la face radiale correspondante de l'anneau intérieur et lesdites faces délimitent un espace de rangement d'un organe de maintien transitoire des corps roulants de support des contraintes radiales à la suite du déplacement axial relatif au boîtier extérieur par rapport à la bague intérieure.

D'autres caractéristiques et avantages de l'invention apparaîtront à la lecture de la description d'exemples de réalisation du roulement :
- la figure 1 est une vue en coupe axiale d'un exemple de réalisation du roulement à deux rangées de corps roulants.
- la figure 2 est une vue en coupe axiale de la bague intérieure du roulement portant une rangée de corps roulants support des contraintes radiales, entourée par l'organe de maintien.
- la figure 3 est une vue en coupe axiale du roulement représenté à la figure 1 au cours du processus d'assemblage du boîtier extérieur avec la bague intérieure.
- la figure 4 est une vue en coupe axiale d'un exemple de réalisation du roulement pour prothèse de hanche qui possède trois rangées de corps roulants et dans lequel le boîtier extérieur possède une surface sphérique.

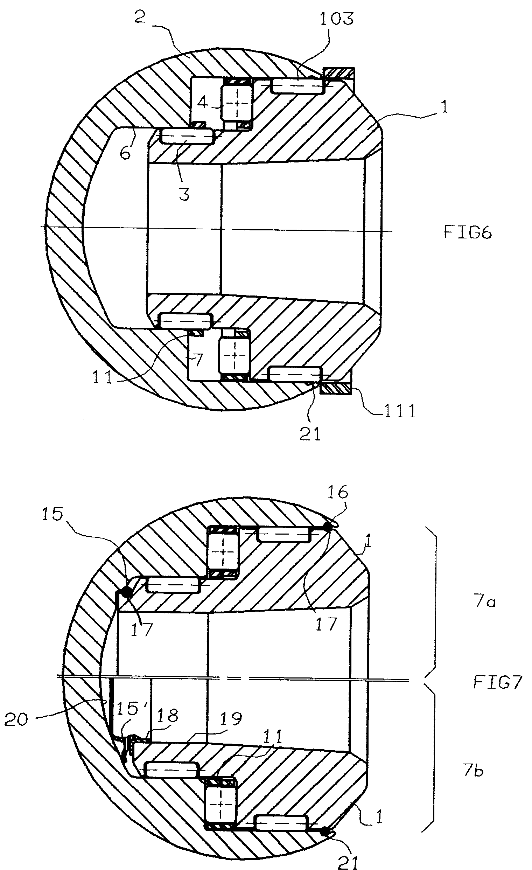

- la figure 5 est une vue en coupe axiale de la bague intérieure du roulement représenté à la figure 4 portant deux rangés de corps roulants support des contraintes radiales, respectivement entourées par un organe de maintien.
- la figure 6 est une vue en coupe axiale du roulement représenté à la figure 4 au cours du processus d'assemblage du boîtier extérieur avec la bague intérieure.
- la figure 7 représente deux demi-vues en coupe axiale de variantes de réalisation étanches du roulement représenté à la figure 4.
- la figure 8 représente deux demi-vues en coupe axiale de la bague intérieure des roulements représentés à la figure 7.
- la figure 9 est une vue en coupe axiale des variantes de réalisation des roulements représentés à la figure 7 au cours des processus d'assemblage des boîtiers extérieurs.
- la figure 10 est une vue en plan de la cage de répartition des organes roulants support des contraintes axiales.
- la figure 11 est une vue en coupe radiale de la cage représentée à la figure 10.

Dans ce qui suit on désignera par les mêmes repères les éléments et organes homologués représentés sur les différentes variantes de réalisation du roulement.

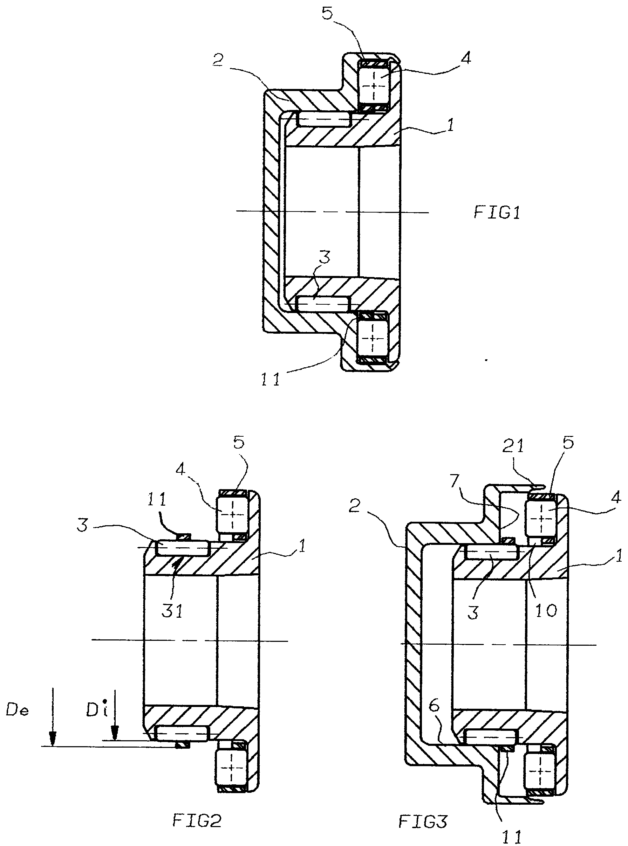

En référence à l'exemple de réalisation du roulement représenté à la figure 1, celui-ci est constitué par une bague intérieure 1, une bague extérieure formant boîtier extérieur 2, des corps roulants 3 support des contraintes radiales, des corps roulants 4 support des contraintes axiales.

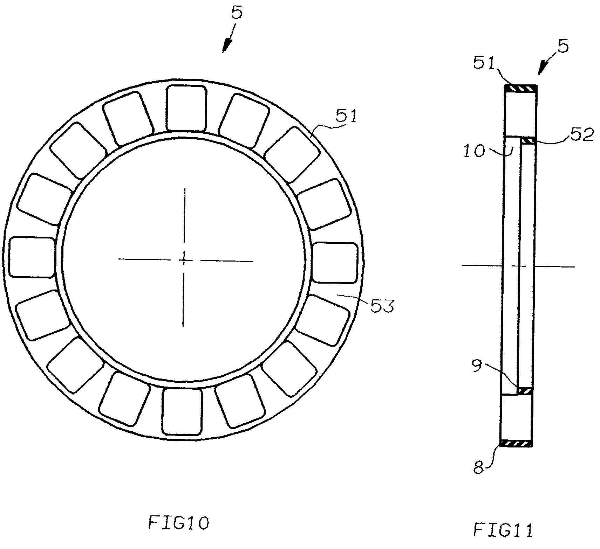

Les corps roulants 4 sont disposés dans une cage 5 représentée plus en détail aux figures 10, 11.

Le boîtier extérieur 2 possède :
- une première surface périphérique intérieure 6 qui constitue le chemin de roulement des corps roulants 3,
- une deuxième surface 7 sensiblement perpendiculaire qui constitue le chemin de roulement des corps roulants 4.

Les corps 4 sont disposés dans la cage 5 constituée ainsi que cela est représenté aux figures 10, 11, par un anneau extérieur 51 et par un anneau intérieur 52 concentriques reliés par des barres radiales 53 d'espacement des corps 4.

Selon l'invention l'anneau extérieur 51 possède une face radiale 8 axialement décalé par rapport à la face radiale correspondante 9 de l'anneau intérieur 52. Selon une particularité de l'invention les faces 8, 9 délimitent un espace 10 de rangement d'un anneau 11 de maintien des organes roulants 3.

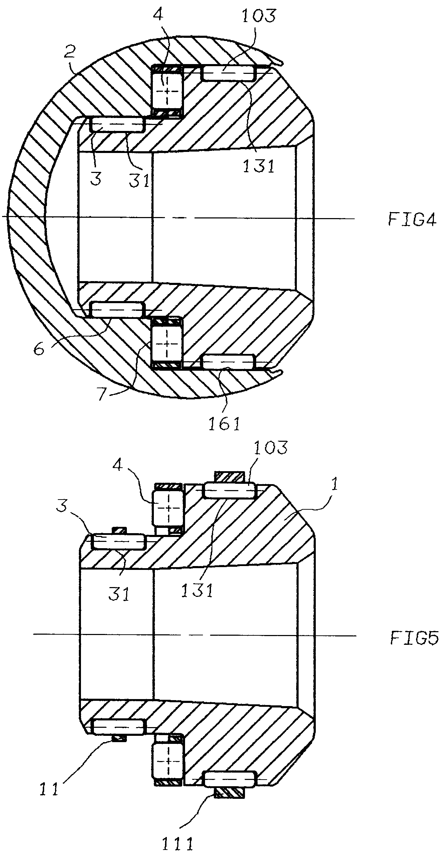

Les figures 2 et 3 montrent que l'anneau 11 assure le'maintien transitoire des corps roulants 3 pendant la phase d'assemblage du roulement au cours de laquelle le boîtier 2 est monté et déplacé axialement sur la bague intérieure 1.

Ainsi que cela est montré à la figure 2, les corps roulants 3 sont disposés dans une gorge 31 portée par la bague 1 et maintenus par l'anneau 11 défini par son diamètre extérieur De et par son diamètre intérieur Di sensiblement égal au diamètre extérieur de la rangée des corps roulants 3.

La figure 3 représente le processus d'assemblage du roulement au cours duquel le boîtier extérieur 2 se centre sur le diamètre extérieur des corps roulants 3 qui sont de la sorte maintenus en place dans la gorge 31 par l'anneau 11.

Une portion circulaire de la surface 7 du boîtier 2 exerce transitoirement une poussée axiale sur l'anneau 11 et ce dernier est placé sous l'effet de la poussée dans l'espace 10 et complète de la sorte la cage 5. Dans le but d'obtenir l'immobilisation axiale du boîtier 2 par rapport à la bague 1, l'ouverture du boîtier possède un bout aminci 21 destiné au sertissage du boîtier au contact de la bague 1.

La figure 4 décrit un exemple de réalisation du roulement plus particulièrement applicable à la réalisation d'une prothèse de hanche.

Dans cet exemple de réalisation, deux rangées de corps roulants 3, 103 sont maintenus dans des gorges 31, 131 de la bague 1 par des anneaux 11, 111.

Selon cet exemple le boîtier 2 possède :
- une première surface périphérique 6 qui constitue le chemin de roulement des corps roulants 3.
- une deuxième surface 7 sensiblement perpendiculaire qui constitue le chemin de roulement des corps roulants 4.
- une troisième surface périphérique 161 co-axiale à la surface 6 qui constitue le chemin de roulement des corps roulants 103.

Au cours du processus d'assemblage représenté à la figure 6 le boîtier extérieur 2 est centré sur le diamètre extérieur des corps roulants 3, 103.

Une portion circulaire de la surface 7 du boîtier 2 exerce, conjointement avec son bord de sertissage aminci 21 transitoirement une poussé axiale sur les anneaux 11, 111.

L'anneau 11 est alors placé comme précédemment dans la cage 5 et l'anneau 111 est éjecté de la bague 1.

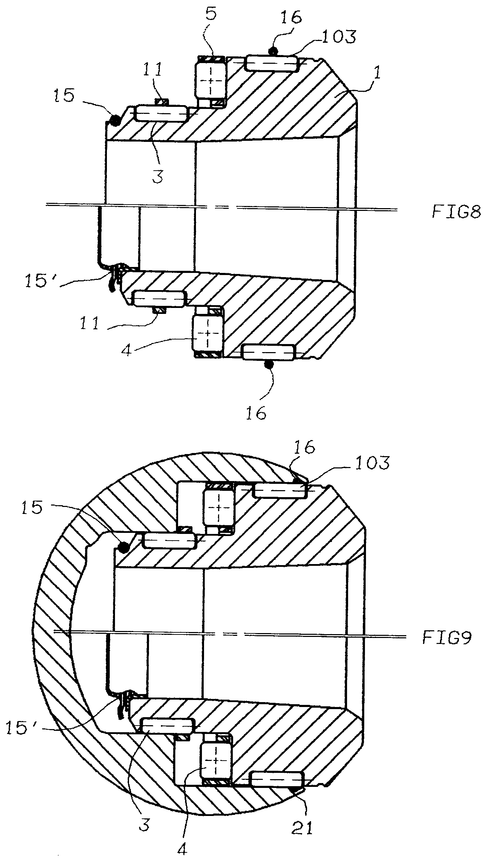

La figure 7 décrit deux exemples de réalisation 7a, 7b d'un roulement étanche applicable à la réalisation de la prothèse.

La bague 1 porte ici des joints d'étanchéité 15, 16 respectivement immobilisés dans des gorges 17 ou sur une portée alésée de la bague.

La partie 7a de la figure 7 montre deux joints d'étanchéité 15, 16 disposés dans les gorges 17.

La partie 7b de la figure illustre un joint 15′ à lèvres axiales radiales monté par son armature 18 dans l'alésage 19 de la bague 1.

Les lèvres du joint 15′ sont en appui sur le fond 20 du boîtier 2.

La partie 7a de la figure illustre un joint torique 15 en appui sur le fond 20 du boîtier 2.

Ainsi que cela est représenté sur la figure 8 l'anneau 11 assure le maintien transitoire des corps roulants 3 sur la bague 1 avant d'être déplacé axialement dans la cage 5.

Le joint d'étanchéité 16 assure le maintien transitoire des corps roulants 103 sur cette même bague.

Le processus d'assemblage représenté à la figure 9 est comparable au processus illustré par la figure 6.

Au cours du processus, l'anneau d'étanchéité 15 est comprimé au contact du fond 20 et l'anneau d'étanchéité 16 est déplacé axialement dans la gorge 17 sous l'action d'une poussée axiale.

Selon cet exemple d'application, le bord 21 du corps 2 recouvre le joint 16 placé dans la gorge 17 et la cohésion du roulement est maintenue par l'élasticité du joint. La combinaison des joints 15, 16 permet d'isoler les différentes parties fonctionnelles du roulement des agents extérieurs et l'emploi de roulements lubrifiés.

Les joints d'étanchéité (15, 15′, 16) seront avantageusement réalisés en un matériau biodégradable tel qu'un polydioxanone. Après installation dans le corps humain d'un tel roulement prélubrifié par le sérum physiologique, les joints d'étanchéité se résorbent et le roulement fonctionne avec le lubrifiant physiologique naturel.

## Revendications

**1°)** Roulement combiné radial-axial à corps roulants (3, 4) montés sur une bague intérieure (1) dans lequel un boîtier extérieur (2) de réception possède une première surface périphérique intérieure (6) qui constitue le chemin de roulement des corps roulants (3, 103) de support des contraintes radiales et une deuxième surface (7) sensiblement perpendiculaire qui constitue le chemin de roulement des corps roulants (4) de support des contraintes axiales, montés dans une cage (5) de réception et de répartition des corps roulants, constituée par un anneau extérieur (51) et un anneau intérieur (52) concentriques et des barres de liaison et d'espacement radiales, caractérisé par le fait que l'anneau extérieur (51) de la cage possède une face radiale (8) axialement décalée par rapport à la face radiale (9) correspondantes de l'anneau intérieur (52) et que lesdites faces délimitent un espace (10) de rangement d'un organe (11) de maintien transitoire de corps roulants de support des contraintes radiales à la suite du déplacement axial relatif du boîtier extérieur (2) par rapport à la bague intérieure (1).

**2°)** Roulement selon la revendication 1, caractérisé par le fait que l'ouverture du boîtier extérieur possède un bord aminci de sertissage (21) et de retenue axiale de la bague intérieure (1).

**3°)** Roulement selon la revendication 2, caractérisé par le fait que le fond (20) du boîtier extérieur (2) et le bord de sertissage (21) de ce dernier sont en appui sur des organes d'étanchéité (15, 15′, 16) portés par la bague intérieure (1).

**4°)** Roulement selon la revendication 3, caractérisé par le fait que lesdits organes d'étanchéité (15, 15′, 16) sont réalisés dans un matériau biodégradable.

**5°)** Roulement selon la revendication 4, caractérisé par le fait qu'il est pré-lubrifié par le sérum physiologique.

**6°)** Roulement selon l'une quelconque des revendications 1 à 5, caractérisé par le fait qu'il constitue une tête fémorale de prothèse de hanche.
